# EUROPEAN PATENT APPLICATION

(11) **EP 4 450 971 A1**
(43) Date of publication of application: **23.10.2024**
(21) Application number: 23169125.4
(22) Date of filing: 21.04.2023
(51) Int. Cl.: G01N 33/533, C12Q 1/6813, G01N 33/532, G01N 33/58

(54) **MARKER FOR ANALYSING A BIOLOGICAL SAMPLE**

(71) Applicant: Leica Microsystems CMS GmbH, 35578 Wetzlar (DE); PixelBiotech GmbH, 69123 Heidelberg (DE); Integrated DNA Technologies Inc., Coralville, IA 52241 (US)
(72) Inventor: Alsheimer, Soeren, 35578 Wetzlar (DE); Cheng, Yongsheng, 69123 Heidelberg (DE); Marvin, Mike, Coralville, 52241 (US)
(74) Representative: Schaumburg und Partner Patentanwälte mbB

(57) **Abstract**

A marker (100) for analysing a biological sample comprises a label (102) with an oligonucleotide backbone (104) and at least a first detectable moiety (106). The marker (100) further comprises an affinity reagent (108) configured to specifically bind to a target molecule (110) of the biological sample. The oligonucleotide backbone (104) of the label (102) is bound to the affinity reagent (108). The oligonucleotide backbone (104) comprises at least one cleavage site (112) cleavable by a chemical agent.

## Description

### Technical field

The invention relates to a marker for analyzing a biological sample. Further, the invention relates to a kit comprising a marker and a method for analyzing a biological sample.

### Background

In the field of spatial biology, multiplexed imaging is an effective technology to examine biological structures, in particular to clearly visualize, identify, and quantify significant biomarkers.

In this technology, it is vital to precisely identify and locate certain structures or target molecules within the biological sample to be examined. This is achieved by introducing markers into the sample that only bind to specific structures, e.g. specific biomolecules. Such a marker typically comprises an affinity reagent that only attaches to the structure in question and a label comprising e.g. one or more fluorescent dyes that are either directly conjugated to the affinity reagent or attached to the affinity reagent by other means, for example a secondary affinity reagent.

Multiplexed imaging serves to identify a large number of markers with high spatial resolution at the same time. Therefore, it is desirable to provide a diverse set of markers having different affinities and labels with varying fluorescent properties.

A typical imaging process involves a staining step in which the markers are applied to a sample where they bind to specific target molecules. Subsequently, a readout is performed in which signals from the labels of the markers are detected. After the readout, the labels are completely or partially removed from the sample so that a next imaging round can be executed. For this purpose, the markers may have cleavage sites which allow the labels to be separated from their markers.

It is still a challenging task to create large libraries of markers that can be used in an imaging process as described above where the labels are to be removed after the readout.

### Summary

It is an object to provide a marker having a label with a detectable moiety that can be easily removed after sample staining.

The afore-mentioned object is achieved by the marker according to claim 1. Advantageous embodiments are defined in the dependent claims and the following description.

A marker is provided that can be used to analyse a biological sample. The marker comprises a label with an oligonucleotide backbone and at least a first detectable moiety. The marker further comprises an affinity reagent configured to specifically bind to a target molecule of the biological sample. The oligonucleotide backbone of the label is bound to the affinity reagent. The oligonucleotide backbone comprises at least one cleavage site cleavable by a chemical agent.

The marker comprises an oligonucleotide-based label that can be used advantageously in imaging processes in which a sample is stained with the label and the same is removed after readout. More specifically, the marker enables label inactivation in multiplexed imaging that is applied e.g. in antibody-based methods. For example, a use case for the marker disclosed herein is a technology known in the field as Cell DIVE which allows the marker to be used with proven cleavage chemistry. Such cleavage/inactivation chemistry is known to be well tolerated by samples over many staining cycles.

The structure of the marker is based on an oligonucleotide backbone. Oligonucleotide-based structures can be used in FISH technology (fluorescence in situ hybridization). A specific example is a platform known in the field as HuluFISH which is a new kind of true multiplex smFISH (sm: single-molecule). The HuluFISH platform allows for tuning brightness of the labels and making combinatorial labels, i.e. labels that include a combination of different detectable moieties. Tunable brightness is helpful for staining targets with high expression and low expression in the same round.

The first detectable moiety of the marker may be a fluorophore, in particular at least one fluorescent molecule, or fluorescent dye that is bound to the backbone and emits fluorescent light when illuminated with excitation light.

The affinity reagent may be configured to specifically bind to a protein, RNA, DNA, metabolite, or micronutrient forming the target molecule that is to be examined.

In a preferred embodiment, the marker may form a fluorescent dye antibody conjugate in which the affinity reagent is an antibody to which a fluorescent dye is bound.

The oligonucleotide backbone may be directly bound to the affinity reagent, for example covalently or mediated by an affinity pair comprising two affinity interactors such as streptavidin and biotin. Alternatively, the oligonucleotide backbone may be indirectly bound to the affinity reagent via hybridization of barcodes. In this case, an oligonucleotide-labeled barcoded affinity reagent may be used.

In an inactivation process, the chemical agent can be used to irreversibly separate the first detectable moiety at the cleavage site formed on the oligonucleotide backbone from the marker. The chemical agent is to be understood as an agent that causes chemical cleavage which in particular is not based on temperature and/or light and is also not an enzymatic cleavage.

According to an embodiment, the chemical agent is at least one of an oxidising agent and a liquid.

The chemical agent may be at least one of a reducing agent such as Dithiothreitol or tris(2-carboxyethyl)phosphine, in which case a cleavable bond is a disulfide bond in the oligonucleotide backbone, and a silver nitrate or other salt having Ag+ ions, in which case a cleavable bond is phosphothioate bond (5'-phosphorothloate linkage 3'-O-P-S-5'). In this respect, reference is made to Mag et al., "Synthesis and selective cleavage of an oligodeoxynucleotide containing a bridged internucleotide 5'-phosphorothioate linkage", Nucl. Acids Res. 19, (1991), 1437-1441. Further reference is made to Perez-Lopez et al., "Synthesis and optimization of a reactive oxygen species responsive cellular delivery system", New J. Chem., (2017), 41, 2392, and Gao et al., "Reactive Oxygen Species Responsive Polymers for Drug Delivery Systems", Front. Chem., Vol. 9, (2021), 649048.

According to a preferred embodiment, the chemical agent may be hydrogen peroxide (H2O2). Hydrogen peroxide can be used advantageously to remove the detectable moiety from the marker in case that the target molecule to be examined is a non-nucleic acid-based molecule. The reason for this is that hydrogen peroxide seems to attack nucleic acid-based molecules while leaving other molecules such as proteins, RNA, DNA, metabolites, or micronutrients less affected or even unaffected.

Regarding the effects of hydrogen peroxide on an oligonucleotide backbone and a potential target molecule, reference is made to Blakeley et al., "Hydrogen Peroxide-Induced Base Damage in Deoxyribonucleic Acid,", Radiation Research; Vol. 121, No. 3 (1990), pp. 338-343. The authors exposed an aqueous solution of calf thymus DNA to hydrogen peroxide in the presence of air and found a number of base modifications including: 8-hydroxyadenine, cytosine glycol, 2,6-diamino-4-hydroxy-5-formamidopyrimidine, 8-hydroxyguanine, thymine glycol, and 4,6-diamino-5-formamidopyrimidine. The authors stated that this damage profile is similar to damage observed after exposure to ionizing radiation which creates radicals. The authors further explained that hydrogen peroxide is thought to participate in a Fenton-like reaction with transition metals, which are readily bound to DNA in trace quantities, resulting in the production of hydroxyl radicals close to the DNA. These results support the role of metal ions bound to DNA in the site-specific formation of highly reactive radical species, most likely hydroxyl radicals, in hydrogen peroxide-induced damage to the bases in DNA.

In the same context, reference is made to Driessens et al., "Hydrogen peroxide induces DNA single- and double-strand breaks in thyroid cells and is therefore a potential mutagen for this organ. Endocrine-related cancer"; Vol. 16, Issue 3 (2009), pp. 845-856. The authors used comet assays to assess the induction of single- and double-strand breaks by hydrogen peroxide, which is produced in cells of the thyroid to oxidize iodide. The authors found that under both alkaline and neutral conditions hydrogen peroxide leads to SSBs/DSBs. A concentration of 0.1mmol/l H2O2 was sufficient to generate a comet assay score of around 245 under alkaline conditions, which was roughly comparable to a gamma-irradiation of 5-10 Gy (scores in the range of 200-300). The effect plateaued with a maximal score of 400 under alkaline conditions and 0.5mmol/l H2O2.

Considering these findings, the alkaline conditions and high concentration of hydrogen peroxide used for dye inactivation with a system such as the afore-mentioned CellDIVE can be expected to not only rapidly bleach a number of fluorescent dyes, but also to breakdown nucleic acid backbones quickly. The strand breaks seem to result primarily from the oxidation of the phosphate bonds of the nucleic acid backbone. Therefore, within the meaning of this disclosure, in particular a phosphate bond of the nucleic acid backbone is in itself a cleavage site that can be cleaved by a chemical agent such as an oxidizing agent, e.g. hydrogen peroxide.

The marker may be formed from a fluorescent dye antibody conjugate as mentioned above. Fluorescent dye antibody conjugates are typically of the direct type, i.e. the dyes are conjugated using NHS-chemistry, maleimide-, or various click chemistries. The inventors realized that conjugation of fluorescent dyes or other detectable moieties via oligonucleotide backbones provides not only numerous advantages with respect to platform flexibility, e.g. in terms of generating combinatorial labels, stoichiometric control, e.g. number of dyes on the label, and tunable brightness as for example in the afore-mentioned HuluFISH technology disclosed in WO2018/060249A1 and WO 2018/188856A1. It also provides a simple and proven way to cleave off labels from affinity reagents by basically shredding their nucleic acid backbones with hydrogen peroxide and washing away the fragments and released detectable moieties.

The cleavage with an oxidizing agent such as hydrogen peroxide is particularly preferred as this allows to apply the same conditions in terms of buffers and protocol that are used in existing systems such as the CellDIVE multiplexed biomarker imaging solution to bleach fluorescent dyes. This is advantageous as a proven and trusted chemistry can be applied that was found to be compatible with most epitopes over as many as ten cycles of staining, imaging, and bleaching. The present solution renders the same protocol and buffers now even more useful and significantly extends the application range to basically any cyclical labeling, readout, label removal process, even outside of microscopy.

The rapid and efficient removal of labels is important in many assays used in the life sciences. This pertains to labels comprising a fluorescent dye, a metal tag, a radioactive moiety, an enzyme that catalyzes reporter generation/deposition and/or generates a detectable signal in another way like luminescence/phosphorescence.

Accordingly, the oligonucleotide backbone may be an oligodeoxyribonucleotide in which each phosphate bond forms a cleavage site for the chemical agent such as hydrogen peroxide. Thus, the chemical agent indiscriminately acts on the phosphate bonds of the oligonucleotide backbone.

According to an alternative embodiment, the oligonucleotide backbone may comprise oligonucleotides which are not cleavable by the chemical agent. For this, rather than natural oligonucleotides, the backbone may comprise non-natural oligonucleotides in which the bonds between the nucleotides cannot be cleaved by the chemical agent.

The cleavage site of the oligonucleotide backbone may comprise at least one cleavage nucleotide which is cleavable by the chemical agent, e.g. hydrogen peroxide. In combination with the afore-mentioned non-natural oligonucleotides which are not cleaved by the chemical agent, the cleavage nucleotide provides a distinct cleavage site as the bond between the cleavage nucleotide and adjacent nucleotides is enabled to be cleaved by the chemical agent.

The cleavage site may be a linker molecule incorporated into the oligonucleotide backbone. In particular, a non-nucleotide-based molecule susceptible to the chemical agent may be incorporated into the oligonucleotide backbone to form the cleavage site.

The label may comprise at least one second detectable moiety such as a fluorescent dye that is bound to the oligonucleotide backbone.

In a specific embodiment, the oligonucleotide backbone comprises at least one further cleavage site cleavable by the chemical agent. The further cleavage site is arranged between the first detectable moiety and the second detectable moiety. The further cleavage site is more susceptible to cleavage by the chemical agent.

It is possible and useful to combine a specific-cleavage site with a first cleaving agent that may or may not be a chemical agent, which offers positional control, with the more generic cleavage site, i.e. the phosphate bonds of the oligonucleotide backbone, responding to the chemical agent such as hydrogen peroxide as a second cleaving agent. Using such a marker enables for example a workflow comprising a sequence of steps of labelling a target molecule, performing a readout, modifying the label, again performing a readout, and then removing the label. Such a workflows is useful if, for example, a modification allows to retrieve positional information about the detectable moieties on the label and may be used to differentiate labels that cannot otherwise be differentiated.

According to another aspect, a kit is provided that comprises a marker and a chemical agent as described above. In a preferred embodiment, an oxidising agent such as hydrogen peroxide is used as chemical agent.

According to another aspect, a method for analysing a biological sample is provided, the method comprising the following steps: a) providing at least a first set of markers as described above, wherein the first set of markers comprises a first plurality of markers with first affinity reagents configured to bind to a first target molecule and each marker comprising a first label; b) introducing at least the first set of markers into the biological sample to allow the markers to bind to their respective target molecule in the biological sample; c) directing excitation light onto the biological sample, the excitation light being configured to visualise at least the first label; and d) generating at least one optical readout from light originating from at least the first label.

According to a preferred embodiment, the first set of markers comprises at least a second plurality of markers with second affinity reagents configured to bind to a second target molecule and each marker comprising a second label.

Preferably, the first label and the second label have different fluorescent properties. These different fluorescent properties can be used to identify the marker, e.g. by irradiating the sample with excitation light of different wavelengths.

According to a further embodiment, the method comprises the following step: e) applying a chemical agent to the biological sample in order to cleave the cleavage site of at least the first set of markers.

Preferably, the method further comprises the following step: f) removing the cleaved off marker.

According to a preferred embodiment, the steps a) to d) are repeated with a second set of markers, wherein the second set of markers comprises at least a third plurality of markers with third affinity reagents configured to bind to a third target molecule.

### Short Description of the Figures

Hereinafter, specific embodiments are described referring to the drawings, wherein:
- Figure 1: is a schematic view of a marker according to an embodiment; and
- Figure 2: is a flowchart showing a method for analysing a biological sample according to an embodiment.

### Detailed Description

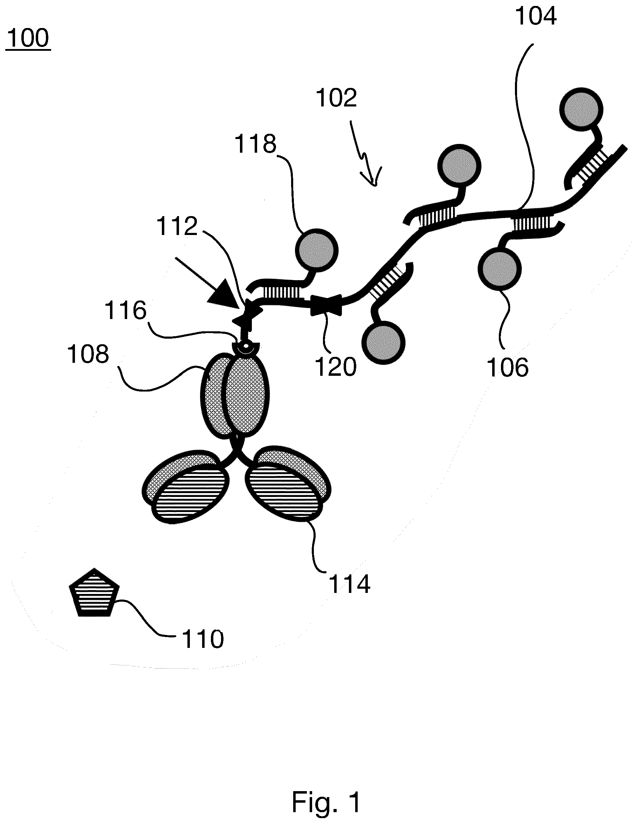

Figure 1 is a schematic view illustrating a marker 100 according to an embodiment.

The marker 100 includes a label 102 having an oligonucleotide backbone 104. The label 102 comprises at least one detectable moiety 106. For instance, the detectable moiety 106 may be formed from a fluorescent dye that is configured to emit fluorescent light when irradiated with suitable excitation light. The label 102 may be provided with one or more fluorescent dye molecules as illustrated in Figure 1. In case of multiple dye molecules, the same type of dye may be used to form a single color label. Alternatively, different types of dyes may be used to form a combinatorial label.

According to the embodiment shown in Figure 1, each first detectable moiety 106 is an oligonucleotide-based entity having a specific nucleotide sequence complementary to the sequence of a specific portion of the oligonucleotide backbone 104. Thus, the first detectable moiety 106 is enabled to bind to the oligonucleotide backbone 104.

The marker 100 further comprises an affinity reagent 108 such as an antibody. The affinity reagent 108 is configured to specifically bind to a target molecule 110 which may be a protein. The specificity of the affinity reagent 108 with respect to the target molecule 110 is shown in Figure 1 by portion 114 illustrated by the same hatching as the target molecule 110. The oligonucleotide backbone 104 of the label 102 is bound to the affinity reagent 108.

The oligonucleotide backbone 104 may be directly bound to the affinity reagent 110, for example by means of direct conjugation 116 via a covalent bond such as a so-called site-click bond, or by means of an affinity pair comprising e.g. streptavidin and biotin. However, it is to be noted that the oligonucleotide backbone 104 may also be indirectly bound to the affinity reagent 108, e.g. through hybridization of an oligonucleotide-labeled barcoded affinity reagent.

The oligonucleotide backbone 104 of the label 102 comprises at least one cleavage site 112 that can be used to inactivate the label 102 by completely or partially removing the label 102 together with the detectable moiety 106 from the affinity reagent 108. To this end, the cleavage site 112 may be configured to be cleavable by means of a chemical agent. For instance, the chemical agent may be an oxidizing agent such as hydrogen peroxide.

In a particular embodiment, the oligonucleotide backbone 104 may be an oligodeoxyribonucleotide. In this case, the at least one cleavage site 112, that is illustrated in Figure 1 by a single site, actually comprises a plurality of cleavage sites. Thus, each phosphate bond of the oligodeoxyribonucleotide forming the oligonucleotide backbone 104 represents a cleavage site which can be cleaved by the chemical agent. Therefore, when the oligonucleotide backbone 104 is formed from an oligodeoxyribonucleotide, the chemical agent indiscriminately acts on substantially all phosphate bonds of the oligonucleotide backbone. Accordingly, the oligonucleotide backbone 104 is basically fragmented by the chemical agent in its entirety. As a result, the fragments of the shredded oligonucleotide backbone 104 can be washed away to inactivate the label 102.

In the afore-mentioned embodiment, the oligonucleotide backbone 104 is formed from natural oligonucleotides which are susceptible to the chemical agent so that the oligonucleotide backbone 104 as a whole is fragmented by the chemical agent. However, the approach disclosed herein is not limited to such a configuration. Rather, in an alternative configuration, the oligonucleotide backbone 104 may comprise non-natural oligonucleotides in which the bonds between the nucleotides are not (or at least less) sensitive to the chemical agent such that these bonds are not cleaved by the chemical reagent.

According to this alternative configuration, the at least one cleavage site 112 shown in Figure 1 may be formed from at least one cleavage nucleotide which is sufficiently sensitive to the chemical agent to be cleaved by the chemical agent. In a preferred embodiment, the cleavage nucleotide representing the cleavage site 112 is susceptible to an oxidizing agent such as hydrogen peroxide.

As the cleavage nucleotide is sensitive to the chemical reagent while the non-natural oligonucleotides substantially forming the structure of the oligonucleotide backbone 104 are not (or at less) sensitive to chemical agent, a unique cleavage site can be provided in the form of the bond between the cleavage nucleotide and adjacent non-natural oligonucleotides.

According to a further alternative embodiment, the at least one cleavage site 112 shown in Figure 1 may be non-nucleotide based molecule susceptible to the chemical agent. For instance, a linker molecule may be incorporated into the oligonucleotide backbone 104 to form the cleavage site 112.

The label 102 may comprise a plurality of different detectable moieties. According to the example shown in Figure 1, in addition the first detectable moiety 106, a second detectable moiety 118 is bound to the oligonucleotide backbone 104. In such case, a further cleavage site 120 may be included in the oligonucleotide backbone 104 with the cleavage site 120 being arranged between the first detectable moiety 106 and the second detectable moiety 118.

Just as an example, the cleavage site 120 may be adapted to be more susceptible to cleavage by the chemical agent than the cleavage site 112. In such a case, the oligonucleotide backbone 104 is fragmented by the chemical agent at the cleavage site 120 and the first detectable moiety 106 is removed while the second detectable moiety 118 remains bound to the affinity reagent 108 and thus to the target molecule 110 when the chemical agent is applied to the sample.

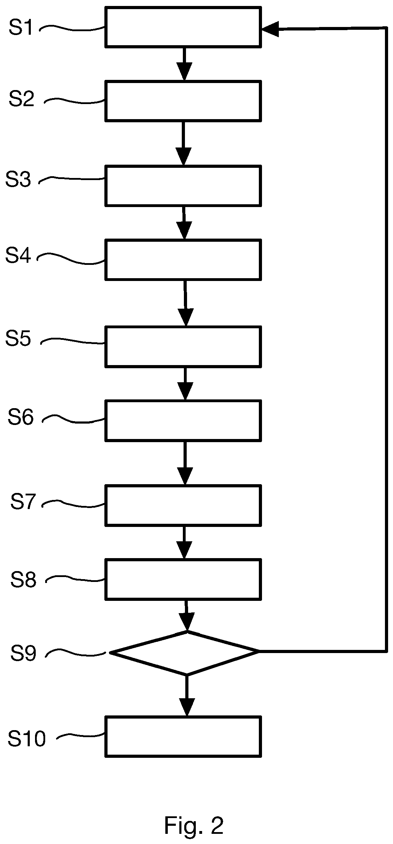

Figure 2 is a flow diagram showing an exemplary method for analysing a biological sample using the marker described above.

In step S1, a first set of markers is provided. The first set comprises a first plurality of markers each having a first label and a first affinity reagent configured to bind to a first target molecule. The first set further comprises a second plurality of markers each having a second label and a second affinity reagent configured to bind to a second target molecule. The first and second affinity reagents are assumed to be of different types. Accordingly, the first and second target molecules are of different types. It is further assumed that the first and second labels have different fluorescent properties.

In step S2, the first set of markers is introduced into the sample. Accordingly, the first plurality of markers is enabled to bind via their first affinity reagents to the first target molecules. Thus, the first target molecules are provided with the first labels. Likewise, the second plurality of markers is enabled to bind via their second affinity reagents to the second target molecules. Thus, the second target molecules are provided with the second labels.

In step S3, the biological sample is irradiated with excitation light that is adapted to cause the first labels of the markers to emit fluorescent light.

In step S4, an optical readout is generated by detecting the fluorescent light originating from the first labels. Thus, the first labels are visualized and/or images thereof might be acquired and stored, in particular for further image analysis.

In step S5, the biological sample is irradiated with excitation light that is adapted to cause the second labels of the markers to emit fluorescent light. Specifically, the wavelength of the excitation light applied in step S5 is different from the wavelength that is used in step S3.

In step S6, an optical readout is generated by detecting the fluorescent light originating from the second labels. Thus, the second labels are visualized and/or images thereof might be acquired and stored, in particular for further image analysis.

In step S7, a chemical agent such as hydrogen peroxide is applied to the biological sample. Accordingly, the cleavage sites formed on the markers are cleaved by the chemical agent. As a result, the first and second pluralities of markers are inactivated.

In step S8, the marker that have been cleaved off in step S7 are removed, e.g. by a washing step.

In step S9, an inquiry is made as to whether or not steps S1 to S8 shall be repeated with a second set of markers.

If the inquiry in step S9 is answered in the affirmative (YES), the process returns to step S1, and the third set of markers is provided. The third set may comprise at least a third plurality of markers each having a third label and a third affinity reagent configured to bind to a third target molecule. The third affinity reagents are assumed to be of a different type than the first and second affinity reagents. Accordingly, the third target molecules are of a different type than the third target molecule.

If the inquiry in step S9 is denied (NO), the process ends in step 510.

The method described above should be understood as only one example of how the marker can be used in spatial biology to study a biological sample. In particular, some of the above steps are only optional such as repeating the process with a second set of markers.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100: marker
- 102: label
- 104: oligonucleotide backbone
- 106: detectable moiety
- 108: affinity reagent
- 110: target molecule
- 112: cleavage site
- 114: portion of affinity reagent
- 116: conjugation
- 118: detectable moiety
- 120: cleavage site

## Claims

1. A marker (100) for analysing a biological sample, comprising:
a label (102) comprising an oligonucleotide backbone (104) and at least a first detectable moiety (106), and
an affinity reagent (108) configured to specifically bind to a target molecule (110) of the biological sample,
wherein the oligonucleotide backbone (104) of the label (102) is bound to the affinity reagent (108),
wherein the oligonucleotide backbone (104) comprises at least one cleavage site (112) cleavable by a chemical agent.

2. The marker (100) according to claim 1, wherein the chemical agent is at least one of an oxidising agent and a liquid.

3. The marker (100) according to claim 1 or 2, wherein the chemical agent is at least one of a reducing agent such as Dithiothreitol or tris(2-carboxyethyl)phosphine, in which case a cleavable bond is a disulfide bond in the oligonucleotide backbone, and a silver nitrate or other salt having Ag+ ions, in which case a cleavable bond is phosphothioate bond (5'-phosphorothloate linkage 3'-O-P-S-5').

4. The marker (100) according to any one of the preceding claims, wherein the chemical agent is hydrogen peroxide.

5. The marker (100) according to any one of the preceding claims, wherein the oligonucleotide backbone is an oligodeoxyribonucleotide.

6. The marker (100) according to any one of the preceding claims 1 to 5, wherein the oligonucleotide backbone (104) comprises oligonucleotides not cleavable by the chemical agent.

7. The marker (100) according to any one of the preceding claims, wherein the cleavage site (112) comprises at least one cleavage nucleotide cleavable by the chemical agent.

8. The marker (100) according to any one of the preceding claims, wherein the cleavage site (112) is a linker molecule incorporated into the oligonucleotide backbone (104).

9. The marker (100) according to any one of the preceding claims, wherein the label (102) comprises at least one second detectable moiety (118).

10. The marker (100) according to claim 9, wherein the oligonucleotide backbone (104) comprises at least one further cleavage site (120) cleavable by the chemical agent, wherein the further cleavage site (120) is arranged between the first detectable moiety (106) and the second detectable moiety (118), and wherein the further cleavage site (120) is more susceptible to cleavage by the chemical agent.

11. A kit comprising a marker (100) according to any one of the preceding claims and a chemical agent.

12. A method for analysing a biological sample, comprising the following steps:
a) providing at least a first set of markers, with markers (100) according to one of the claims 1 to 10, wherein the first set of markers comprises a first plurality of markers with first affinity reagents configured to bind to a first target molecule and each marker comprising a first label,
b) introducing at least the first set of markers into the biological sample to allow the markers to bind to their respective target molecule (104) in the biological sample,
c) directing excitation light onto the biological sample, the excitation light being configured to visualise at least the first label, and
d) generating at least one optical readout from light originating from at least the first label.

13. The method according to claim 12, wherein the first set of markers comprises at least a second plurality of markers with second affinity reagents configured to bind to a second target molecule and each marker comprising a second label.

14. The method according to claim 13, wherein the first label and the second label have different fluorescent properties.

15. The method according to any one of the claims 12 to 14, further comprising the following step:
e) applying a chemical agent to the biological sample to cleave the cleavage site of at least the first set of markers.

16. The method according to claim 15, further comprising the following step:
f) removing the cleaved off marker.

17. The method according to claim 15 or 16, wherein the steps a) to d) are repeated with a second set of markers, wherein the second set of markers comprises at least a third plurality of markers with third affinity reagents configured to bind to a third target molecule.
